# EUROPEAN PATENT APPLICATION

(11) **EP 1 994 983 A1**
(43) Date of publication of application: **26.11.2008**
(21) Application number: 07009638.3
(22) Date of filing: 14.05.2007
(51) Int. Cl.: B01J 23/44, B01J 37/02, B01J 37/30, C07C 69/612, C07C 69/618

(54) **Catalyst containing covalently bonded formate groups and Pd(0) and process for its obtention**

(71) Applicant: Almquest AB, 904 20 Umeå (SE)
(72) Inventor: Almqvist, Fredrik, 904-20 Umeå (SE); Basu, Basudeb, Siliguri, DT. Darjeeling 734 403 (IN)
(74) Representative: Bergstrand, Mikael Gudmundsson

(57) **Abstract**

1. A method of producing a heterogeneous catalyst suitable for catalyzing Heck, Suzuki-Miyaura and Buchwald-Hartwig reactions, comprising the steps of:
a) providing a porous carrier, said porous carrier consisting of a core and a plurality of ion exchange groups covalently bonded to the surface of said core, where at least 90 % of the ions bound to said carrier are formate ions;
b) providing a palladium (II) salt;
c) suspending said carrier in an organic solvent thereby obtaining a suspension;
d) adding said palladium salt to said suspension and allowing the resulting mixture to react at a temperature within the range of 30 - 70 °C until said carrier has turned black;
e) washing said carrier in water; and
f) drying said carrier under vacuum;

characterised in that
the resulting carrier subsequently is resuspended in dimethyl formamide and heated under inert atmosphere to at least 90 °C for 2 hours followed by washing with a polar solvent and drying.

The invention also relates to a catalyst produced by the method and to processes where the catalyst is used for catalysing Heck, Suzuki-Miyaura, and Buchwald-Hartwig reactions.

## Description

The present invention relates to a new catalyst which is suitable for catalysing Heck, Suzuki-Miyaura, and Buchwald-Hartwig reactions. The invention also provides a method for producing the catalyst as well as processes wherein the catalyst is used for catalysing Heck, Suzuki-Miyaura, and Buchwald-Hartwig reactions.

### Technical background

Transition metal-catalyzed organic reactions constitute the central part of contemporary organic synthesis. In particular, palladium-catalyzed carbon-carbon and carbon-heteroatom (N and O) bond-forming processes represent the foremost reactions in the arena of organic process development (All documents cited in this description are incorporated by reference unless nothing else is stated: (a) Organopalladium Chemistry for Organic Synthesis (Ed.: E.-i. Negishi), Wiley-Interscience, New York, 2002; b) Metal-Catalyzed Cross-Coupling Reactions (Eds.: A. de Meijere, F. Diederich), Wiley-VCH, Weinheim, 2004; c) C. C. Mauger, G. A. Mignani, Aldrichimica Acta.2006, 39, 17-24; d) W. A. Herrmann, K. Öfele, D. v. Preysing, S. K. Schneider, J. Organocmet. Chem. 2003, 687, 229-248.) While the Heck, Suzuki-Miyaura, Sonogashira reactions are excellent tools for carbon-carbon coupling reactions between aryl halides or triflates and suitable partners, Buchwald-Hartwig developed the expeditious means for carbon-heteroatom (N and O) bond-forming reactions (W. A. Herrmann, K. Öfele, D. v. Preysing, S. K. Schneider, J. Organocmet. Chem.2003, 687, 229-248.). Over the last decade, commendable research has been done to achieve significant developments and to establish practical methodologies. Widespread uses of the palladium-catalyzed coupling reactions are found in modem organic synthesis, because the resulting coupled products often find good applications in the preparation of materials ( a) N. Miyaura, Adv. Metal-Org. Chem.1998, 6, 187-243; b) L. F. Tietze, G. Kettschau, U. Heuschert, G. Nordman, Chem. Eur. J. 2001, 7, 368-373), pharmaceuticals (a) For review on recent applications of Suzuki-Miyaura coupling, see: S. Kotha, K. Lahiri, D. Kashinath, Tetrahedron2003, 58, 9363-9695; b) For review on Heck coupling, see: I. P. Beletskaya, A. V. Cheprakov, Chem. Rev.2000, 100, 3009-3066), and other bioactive compounds (a) Nicolaou, K. C.; Li, H.; Boddy, C. N. C.; Ramanjulu, J. M.; Yue, T.-Y.; Natarajan, S.; Chu, X.-J.; Brase, S.; Rubsam, F. Chem. Eur. J. 1999, 5, 2584-2601; b) Kamikawa, K.; Watanabe, T.; Daimon, A.; Uemura, M. Tetrahedron2000, 56, 2325; c) A. Haberli, C. J. Leumann, Org. Lett. 2001, 3, 489-492).

Since the common facet of these homo- and hetero-coupling reactions is the palladium, which is believed to act as the catalyst, numerous attempts have been made to employ it either as soluble homogeneous (a) Applied Homogeneous Catalysis(Eds.: B. Cornils, W. A. Herrmann), Wiley-VCH, Weinheim, 1998; b) H. -U. Blaser, A. Indolese, A. Schnyder, H. Steiner, M. Studer, J. Mol. Catal. A2001, 173, 3-18; d) K. C. Nicolaou, A. E. Koumbis, M. Takayanagi, S. Natarajan, N. F. Jain, T. Bando, H. Li, R. Hughes, Chem. Eur. J. 1999, 5, 2622-2647; e) M. T. Reetz, E. Wastermann, Angew. Chem., Int. Ed. 2000, 39,165-168; f) A. Zapf, M. Beller, Chem. Eur. J.2001, 7, 2908-2915.) or as immobilized heterogeneous Pd-catalysts (a) J. Schwarz, V. P. W. Böhm, M. G. Gardiner, M. Grosche, W. A. Herrmann, W. Heiringer, G. Raudaschl-Seiber, Chem. Eur. J. 2000, 6, 1773-1780. b) N. -H. Nam, S. Sardari, K. Parang, J. Comb. Chem.2003, 5, 480-546; c) A. Biffis, M. Zecca, M. Basato, J. Mol. Cat. A: Chem. 2001, 173, 249-274; d) J. Zhou, R. Zhou, L. Mo, S. Zhou, X. Zheng, J. Mol. Cat. A: Chem.2002, 178, 289-292; e) J. A. Gladysz (Ed.) Chem. Rev.2002, 102, 3215-3892; f) S. V. Ley, I. R. Baxendale, R. N. Bream, P. S. Jackson, A. G. Leach, D. A. Longbottom, M. Nesi, J. S. Scott, I. Storer, S. J. Taylor, J. Chem. Soc., Perkin Trans. 1 2000, 3815-4195). Whereas the soluble homogeneous Pd-catalysts are found to be significantly active in various coupling reactions, there are some drawbacks such as, less active or inactive colloidal species, unrecoverable and possibility of contamination with the products, cost etc. that are often encountered in the course of the reactions ( a) W. A. Herrmann, C. Brosner, K. Ofele, M. Beller, H. Fischer, J. Mol. Catal. A: Chem. 1995, 103, 133-146, b) M. Beller, H. Fischer, K. Kuhlein, C. P. Reisinger, W. A. Herrmann, J. Organomet. Chem.1996, 520, 257-259). Consequently, several approaches have been explored utilizing various immobilization techniques on solid or colloidal supports, and aiming toward efficient recovery and reuse of the active catalysts. The major thrust for achieving success in designing and developing polymer-supported metal catalysts broadly include: improved stability within the polymer matrix, increased selectivity for reactions, enhanced regioselectivity, reusability for several runs and superior asymmetric induction due to site-specific chiral catalysts. Attempts have been made to surmount the problems pertaining to high reaction temperatures, separation of pure products, recovery and reuse of Pd-complexes by the use of heterogeneous palladium systems. Pd on carbon and Pd on different metal oxides (a) A. Eisenstadt In Utilization of the Heterogeneous Palladium-on-Carbon Catalyzed Heck Reaction in Applied Synthesis, Ed.: F. E. Herkes; Marcel Dekker, Basel, 1998; Vol 1, pp 415-427; b) F. Zhao, B. M. Bhanage, M. Shirai, M. Arai, Chem. Eur. J. 2000, 6, 843-848; c) M. Wagner, K. Köhler, L. Djakovitch, S. Weinkauf, V. Hagen, M. Muhler, Top. Catal. 2000, 13, 319-326; d) G. W. Kabalka, V. Namboodiri, L. Wang, Chem. Commun.2001, 775; e) H. Sakurai, T. Tsukuda, T. Hirao, J. Org. Chem. 2002, 67, 2721-2722; f) T. Tagata, M. Nishida, J. Org. Chem.2003, 68, 9412-9415; g) M. Gruber, S. Chouzier, K. Köhler, L. Djakovitch, Appl. Catal. A2004, 265, 161-169), silica (a) B. Pawelec, R. Mariscal, R. M. Navarro, S. van Bokhorst, S. Rojas, J. L. G. Fierro, Appl. Catal. A. 2002, 225, 223-237; b) C. P. Mehnert, J. Y. Ying, Chem. Commun.1997,2215-2216; c) J. H. Clark, D. J. Macquarrie, E. B. Mubofu, Green Chem.2000, 2, 53-56), zeolites (a) L. Djakovitch, K. Kohler, J. Am. Chem. Soc.2001, 123, 5990-5999; b) A. Corma, H. Garcia, A. Leyva, A. Primo, Appl. Catal. A: Gen.2003, 247, 41-49; c) L. Artok, H. Bulut, Tetrahedron Lett.2004, 45, 3881-3884; d) S. S. Pröck1, W. Kleist, M. A. Gruber, K. Köhler, Angew. Chem.2004, 116, 1917-1918*;* Angew. Chem., Int. Ed.2004, 43, 1881-1882), molecular sieves (C. P. Mehnert, D. W. Weaver, J. Y. Ying, J. Am. Chem. Soc. 1998, 120, 12289-12296), hydrotalcite (T. H. Bennur, A. Ramani, R. Bal, B. M. Chanda, S. Sivasankar, Catal. Commun. 2002, 3, 493-496) and sepiolites (a) A. Corma, H. Garcia, A. Leyva, A. Primo, Appl. Catal. A: Gen.2004, 257, 77-83; b) K. Shimizu, T. Kan-no, T. Kodama, H. Hagiwara, Y. Kitayama, Tetrahedron Lett.2002, 43, 5653-5655; c) K. Shimizu, R. Maruyama, S. Komai, T. Kodama, Y. Kitayama, J. Catal. 2004, 227, 202-209) were found to be suitable catalysts for some reactions. Several groups have examined use of polymer/dendrimer supported Pd-catalysts, where the immobilization of palladium has been achieved through covalent and non-covalent linkages and using coordinating phosphine ligands attached to the polymeric framework (For recent representative examples, see: a) D. E. Bergbreiter, P. L. Osburn, Y. -S. Liu, J. Am. Chem. Soc.1999, 121, 9531-9538; b) Y. Uozumi, H. Danjo, T. Hayashi, J. Org. Chem.1999, 64, 3384-3388; c) W. -C. Shieh, R. Shekhar, T. Blacklock, A. Tedesco, Synth. Commun.2002, 32, 1059-1067; d) C. A. McNamara, F. King, M. Bradley, Tetrahedron Lett.2004, 45, 8239-8243; e) A. N. Cammidge, N. J. Baines, R. K. Bellingham, Chem. Commun.2001, 2588-2589; f) Y. M. A. Yamada, K. Takeda, H. Takahashi, S. Ikegami, Org. Lett. 2002, 4, 3371-3374; g) Y. M. A. Yamada, K. Takeda, H. Takahashi, S. Ikegami, J. Org. Chem. 2003, 68, 7733-7741). There has been significant interest in the use of supported nanopalladium complexes as catalysts for C-C coupling reactions ( M. Moreno-Mafias, R. Pleixats, Acc. Chem. Res. 2003, 36, 638-643; A. Roucoux, J. Schulz, H. Patin, Chem. Rev.2002, 102, 3757-3778; B. M. Choudary, S. Madhi, N. S. Chowdari, M. L. Kantam, B. Sreedhar, J. Am. Chem. Soc.2002, 124, 14127-14136; D. Astruc, F. Lu, J. R. Aranzaes, Angew. Chem.2005, 117, 8062-8083; Angew. Chem., Int. Ed.2005, 44, 7852-7872; M. Studer, H. -U. Blaser, C. Exner, Adv. Synth. Catal. 2003, 345, 45-65; L. Wu, B. -L. Li, Y. -Y. Huang, H. -F. Zhou, Y. -M. He, Q. -H. Fan, Org. Lett. 2006, 8, 3605-3608). More recently, heterogeneous Pd-catalysts have been developed through microencapsulation technology, optionally with activating ligands, within a highly cross-linked polyurea matrix (a) C. Ramarao, S. V. Ley, S. C. Smith, I. M. Shirley, N. De Almerda, Chem. Commun.2002, 1132-1133; b) J. Q. Yu, H. C. Wu, C. Ramarao, J. B. Spencer, S. V. Ley, Chem. Commun. 2003, 678-679; c) S. V. Ley, C. Mitchell, D. Pears, C. Ramarao, J. Q. Yu, W. Zhou, Org. Lett. 2003, 5, 4665-4668; d) V. Chechik, R. M. Crooks, J. Am. Chem. Soc.2000, 122, 1243-1244), polystyrene (a) R. Akiyama, S. Kobayashi, Angew. Chem.2001, 113, 3577-3579; Angew. Chem., Int. Ed.2001, 40, 3469-3471; b) S. Kobayashi, R. Akiyama, Chem. Commun.2003, 449-460), cyclodextrine (CD) (L. Strimbu, J. Liu, A. E. Kaifer, Langmuir 2003, 19, 483-485), and other dendrimer/polymer surface (E. H. Rahim, F. S. Kamounah, J. Frederiksen, J. B. Christensen, Nano Lett.2001, 1, 499-501; M. Pittelkow, K. Moth-Poulsen, U. Boas, J. B. Christensen, Langmuir 2003,19, 7682-7684; J. C. Garcia-Martinez, R. Lezutekong, R. M. Crooks, J. Am. Chem. Soc.2005, 127, 5097-5103). Advantages of such polymer-supported Pd-catalysts are focused with emphasis on several factors e.g. very low residual metal and ligand level in final products, easy of recovery by filtration, re-use and recycling, high activity, selectivity and compatibility. While some of these supported catalysts exhibit excellent activity and selectivity in various reactions, most of them suffer from general compliance with all the conditions and often require difficultly accessible and well-designed polymeric frameworks. Therefore, procedural complexity and versatile applicability have remained seemingly important and major thrust for further investigations in this field of research.

Very recently, we reported the first heterogeneous Pd-catalyst, immobilized as Pd⁽⁰⁾ on a surface of ion-exchange resins (B. Basu, S. Das, P. Das, A.K. Nanda, Tetrahedron Lett. 2005, 46, 8591-8593). The Amberlite^{®} resins (chloride form) was first exchanged with formate anion to prepare the Amberlite resin formate (symbolized as ARF), which was then treated with catalytic amounts of Pd(OAc)₂ in dimethyl formamide (DMF). The Pd^{(II)} species was soon found to be reduced to Pd⁽⁰⁾ in presence of formate anion present as the counter anion of the Amberlite resins. The grayish beads of ARF were turned black, probably with the deposition of Pd⁽⁰⁾ on the resin surface. Since the resulting Amberlite resin formate soaked palladium complexes possess both the reducing source in presence of suitable metal catalyst (here the formate anion and the palladium), we envisaged use of these complexes in catalytic transfer hydrogenation. Indeed, the complexes showed good efficiency in catalytic transfer hydrogenation of functionalized C-C or C-heteroatom (N and O) double bonds.

However, it was also clear that the complexes were not sufficiently stable and that there was an unacceptably high leakage of palladium from the beads. Accordingly, there is a need for an improved catalyst were the problems regarding stability and leakage have been solved.

### Summary of the invention

In a first aspect, the present invention provides a method of producing an improved heterogeneous catalyst suitable for catalysing Heck, Suzuki-Miyaura and Buchwald- Hartwig reactions, where the above mentioned problems have been solved. Said method is disclosed in claim 1.

Accordingly, the method involves using a porous carrier, said porous carrier consisting of a core and a plurality of ion exchange groups covalently bonded to the surface of said core, where at least 90 % of the ions that are bound to the beads are formate ions. Preferably, at least 95 % of the ions bound to the beads are formate ions. Most preferably, at least 99 % of the ions that are bound to the beads are formate ions.

Typically, the carrier could have the shape of a spherical particle. It could also typically have a size ranging from 10 µm to 2 mm and a porosity from 50 to 1000 Å. Alternatively, the carrier could have a monolithic structure. The core of the carrier is typically made of a material chosen from the group of porous silica, zirconium, graphite, or a polymer or copolymer chosen from the group of mono- or oligovinyl monomer units, such as styrene and its substituted derivatives, acrylic acid or methacrylic acid, alkyl acrylates and methacrylates, hydroxyalkyl acrylates and methacrylates, acrylamides and methacrylamides, vinylpyridine and its substituted derivatives, divinylbenzene, divinylpyridine, alkylene diacrylate, alkylene dimethacrylate, oligoethylene glycol diacrylate and oligoethylene glycol dimethacrylate with up to 5 ethylene glycol repeat units, alkylene bis(acrylamides), piperidine bis(acrylamide), trimethylolpropane triacrylate, trimethylolpropane trimethacrylate, pentaerythriol triacrylate and tetraacrylate, and mixtures thereof.

As mentioned above, ion-exchange groups have been introduced on the surface of said carrier core by functionalizing via a chemical reaction. In a preferred embodiment, the ion-exchange groups have been introduced to the carrier by polymerisation, preferably graft polymerisation onto the surface of said carrier core. Preferably, the ion exchange groups are type 1 or type 2 quaternary ammonium groups.

It is especially preferred that the carrier is an Amberlite IRA bead, such as Amberlite IRA-420 (BDH, GB) or Amberlite IRA-900 (Across, BE).

Furthermore, the method involves using a palladium (II) salt. In principle, any palladium (II) salt could be used in the method. Palladium acetate (Pd(CH₃COO)₂) and disodium palladium tetrachloride (Na₂PdCl₄)are preferred.

Said beads are suspended in an organic solvent. Most organic solvents could be used. Preferably, the organic solvent is chosen from the group of dimethyl formamide (DMF), acetonitrile, toluene and dioxane. Dimethyl formamide is especially preferred. Said palladium salt is then added to said suspension and the resulting mixture is allowed to react at a temperature within the range of 30 - 70 °C, and preferably within the range of 40 - 60 °C. A temperature within the range of 45 - 55°C is especially preferred. The reaction is deemed to be completed when said beads have turned black. The black colour is associated with deposition of Pd(0). The beads are washed in water and dried under vacuum. Subsequently, the beads are resuspended in dimethyl formamide, and heated under an inert atmosphere to at least 90°C, preferably at least 95°C, for 2 hours followed by washing with a polar solvent chosen from the group of dimethyl formamide, water dichloromethane, acetone, diethyl ether, tetrahydrofuran or dioxane and finally drying.

It is preferred that resulting carrier during the last heat treatment at 90 °C under inert atmosphere is allowed to catalyse a chemical reaction chosen from the group of Heck, Suzuki-Miyaura and Buchwald-Hartwig reactions, thereby further improving stability.

In a preferred embodiment, carriers such as Amberlite beads to which formate ions have been bound can be prepared by exposing the chloride form of Amberlite resin, such as Amberlite IRA-420 (BDH, GB) or Amberlite IRA-900 (Across, BE) to formic acid until release of chloride ions from the resin no longer can be observed.

In a second aspect, the present invention also provides a heterogeneous catalyst comprising a porous carrier consisting of a core and a plurality of ion exchange groups covalently bonded to the surface of said core, typically Amberlite IRA-420 or Amberlite IRA-900, on which surface Pd(0) as well as formate has been deposited. Said catalyst has been produced by a method according to the first aspect of the present invention. Below, said catalyst based on Amberlite IRA-420 and/or Amberlite IRA-900 is also referred to as ARF-Pd.

In a third aspect, the present invention provides a reactor comprising a hollow tube having two open ends, which reactor contains a heterogeneous catalyst according to said second aspect.

In a fourth aspect, the present invention relates to using said catalyst for catalysing Heck, Suzuki-Miyaura, and Buchwald- Hartwig reactions.

### Experimental part

The invention will now be described with reference to the enclosed examples and figures.

Figure 1 presents a ¹³C MAS NMR of ARF.

Figure 2 shows a ¹³C MAS NMR of ARF-Pd.

Figure 3 displays SEM micrograph images (10000x magnified) of ARF and ARF-Pd.

Figure 4 discloses XPS survey spectrum and Pd 3d spectrum of the precatalyst ARF-Pd.

Figure 5 reveals a Pd 3d spectrum of fresh ARF-Pd precatalyst prepared from Pd(OAc)₂.

Figure 6 discloses a diagram showing the Pd/NH ratio of the catalyst as a function of the amount of catalytic cycles.

The examples should not be construed as limiting the scope of the appended claims.

### Example 1: Catalyst preparation

Amberlite^{®} IRA 900 (Chloride form) (Across, BE) was packed in a column and treated with 10% aqueous formic acid solution repeatedly until a negative response was observed for chloride ion. The resin was then washed several times with water and dried under vacuum (B. Basu, Md. M. H. Bhuiyan, P. Das and I. Hossain, Tetrahedron Lett.2003, 44, 8931-8934). Palladium acetate (15 mg, 0.067 mmol) was added to a suspension of ARF (2 g) in DMF (4 ml) and the mixture was stirred under N₂ for 1 h at room temperature and then at 50 °C for 3 hr. The orange or dark orange color of the solution became totally colorless, whereas the ARF beads became black. Such color changes were not observed while using the Amberlite^{®} IRA 900 (Chloride form) and Pd(OAc)₂ or Na₂PdCl₄ in control experiments, which led to suggest that Pd^{(II)} species were reduced to Pd⁽⁰⁾ in presence of the counter anion (formate as the reducing source) and then soaked on the resin surface. The grayish beads were turned black and the dark orange supernatant became totally colorless by this time. The mixture was cooled, filtered off the resin beads and washed with water until the washing was almost free from sodium (≤0.1 mg per liter of water; analyzed by flame photometer) (applicable when Na₂PdCl₄ was used). The black resins were then dried under vacuum (50°C / 0.1 mm of Hg).

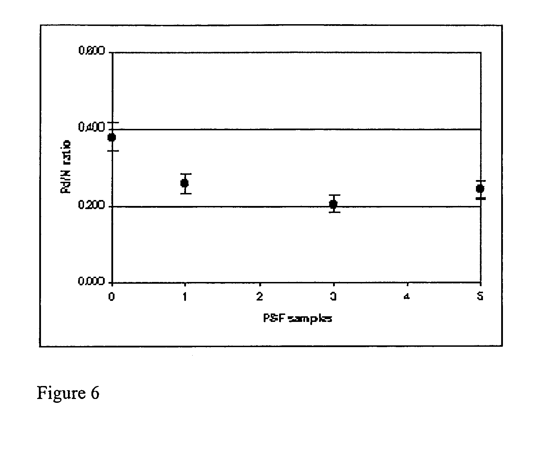

In order to enhance stability, the resulting ARF-Pd was again taken in DMF (4 ml) and heated under N₂ at 100 °C for 2 hr. After cooling, the beads were filtered off and washed with dichloromethane and dried under vacuum. A mixture of 1,3-dibromobenzene (236 mg, 1.0 mmol), phenylboronic acid (271 mg, 2.2 mmol) and sodium carbonate (424 mg, 4 mmol) was taken in DMF (2 ml) and then added to 300 mg of the ARF-Pd beads (300 mg, 1 mol% Pd). The mixture was heated under nitrogen at 110 °C for 2.5 h. After cooling, the reaction mixture was diluted with 10 ml of cold water and filtered through a cotton bed. The ARF-Pd thus obtained (Cycle 1, in figure 6) could be used in several catalytic cycles without any significant change in Pd/N ratio and catalytic activity.(Cycle 3 and cycle 5 in figure 6). Such a ARF-Pd therefore shows an unexpectedly high stability. A catalyst prepared without carrying out the above disclosed step (Sample 0 in figure 6) has good catalytic activity but does not retain its Pd/N ratio indicating leakage of palladium and, accordingly, a lesser degree of stability.

### Example 2: Catalyst preparation starting from Na₂PdCl₄

The method of example 1 was repeated but an equal molar amount of Na₂PdCl₄ was used instead of Pd(OAc)₂. A similar result was obtained.

### Example 3: FT-IR spectroscopy of the ARF-Pd

The ARF-Pd was first characterized by IR spectroscopy. Carboxylic acid salts are most accurately represented with the resonance stabilized carboxylate anions that contain two identical C-̅ -̅ -̅O bonds, and therefore give anti-symmetric and symmetric stretching absorptions. The FT-IR spectral data for the carboxylate anion of different formate salts, ARF and the ARF-Pd are given in Table 1. The spectrum of ARF-Pd was compared with those of ammonium formate (HCOONH₄), potassium formate (HCOOK) and the ARF. The absorptions due to anti-symmetric stretching of the carboxylate anions of HCOONH₄, HCOOK and ARF were observed in the range of 1595-1593 cm⁻¹, while that of ARF-Pd at 1653 cm⁻¹. Similar observations were noticed in the case of symmetric stretching of the carboxylate anions. The significant increase of νₘₐₓ for ARF-Pd indicated possible deposition and binding of the palladium metal with the formate carbonyl species.

**Table 1: FT-IR data for the carboxylate anion (KBr)**

| Entry | Symmetric Stretching (□ₘₐₓ) Cm ⁻¹ | Anti-symmetric Stretching (□ₘₐₓ) Cm⁻¹ |
|---|---|---|
| HCOONH₄ | 1354 | 1595 |
| HCOOK | 1348 | 1593 |
| ARF | 1344 | 1593 |
| ARF̃Pd | 1404 | 1653 |

### Example 4: ¹³C MAS NMR spectroscopy

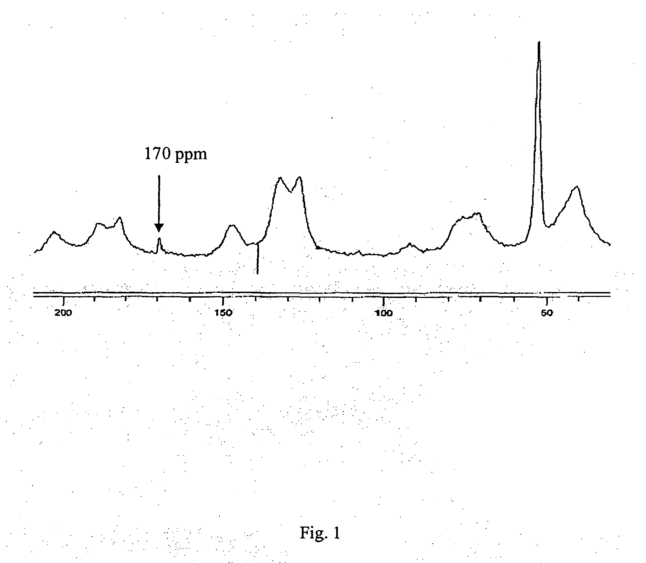

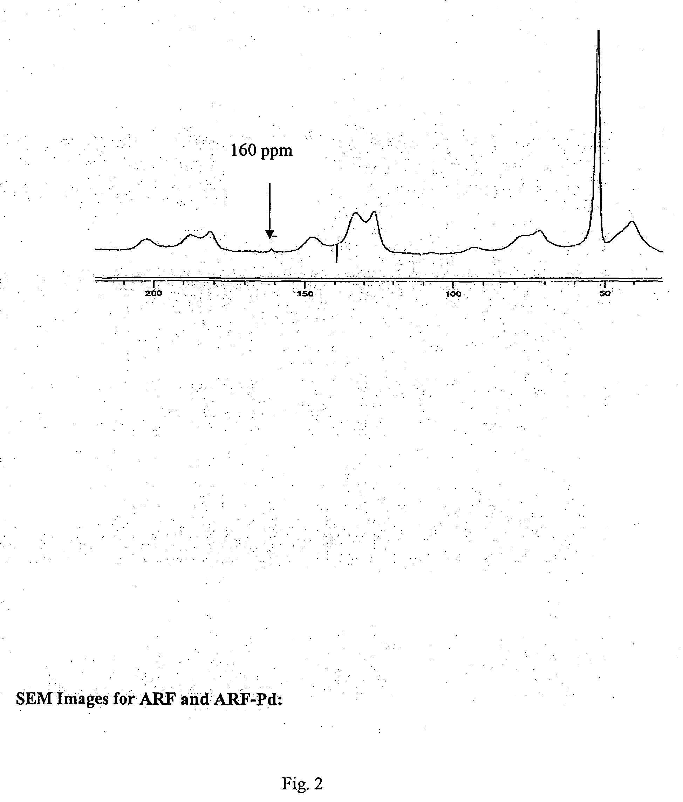

The MAS ¹³C NMR spectra of ARF (Figure 1)and ARF-Pd (Figure 2) are recorded with special attention to the δ position of the carbonyl carbon (formate). As compared to the formate carbonyl carbon of ARF, the same has shown nearly δ 10 ppm upfield shift for ARF-Pd. Thus, the carbonyl carbon in ARF appeared at about δ 170 ppm (Figure 1) while that of ARF-Pd displayed nearly at δ 160 ppm (Figure 2). Such upfield shifting in ARF-Pd may be attributed to binding of palladium with the formate carbonyl group.

### Example 5: Scanning Electron Micrographs

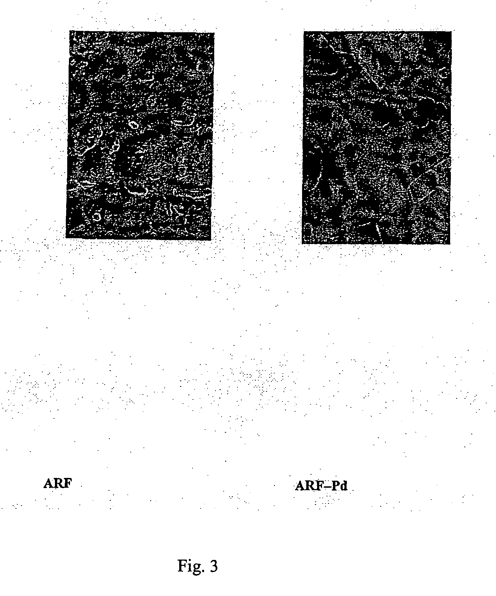

Scanning electron micrographs of ARF and ARF-Pd were examined at 10000x magnification using JEOL JSM 5700F scanning electron microscope. The SEM images (Figure 3) illustrate clearly the differences in surface and shape characteristics and one might explain that palladium has been deposited in the surface of the resins.

### Example 6: XPS studies

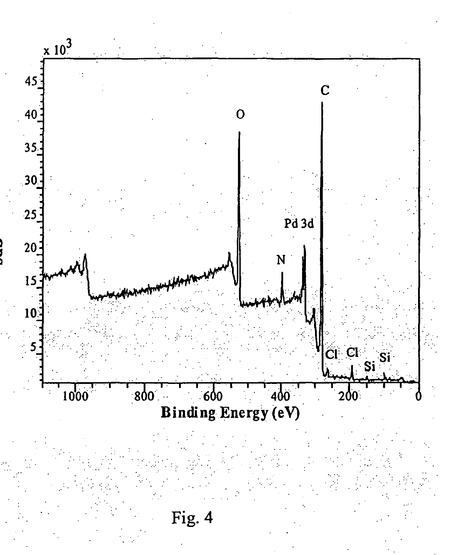

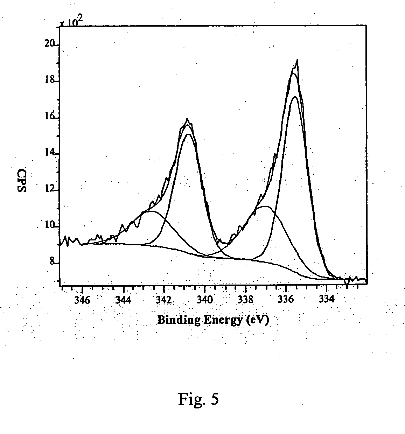

The presence of Pd in the surface was confirmed by XPS survey spectra (Figure 4). Binding energy (BE) of Pd 3d line indicates that the main component of palladium is in the metallic form (335.7 eV), while the minor part may be assigned to Pd^{(II)} cation (337.8 eV). Two lines for Pd⁽⁰⁾ [335.7 eV (3d_{5/2}) and 340.8 (3d_{3/2}) eV], which are observed in the XPS spectrum of ARF-Pd, conform to the observations reported in other related studies (B. M. Choudary, S. Madhi, M. L. Kantam, B. Sreedhar, Y. Iwasawa, J. Am. Chem. Soc.2004, 126, 2292-2293). Minor presence of Pd^{(II)} species might be possible from the fact that the Pd^{(II)} salts were not completely reduced during the preparation of ARF-Pd. Comparison of the spectra of ARF-Pd prepared from Pd(OAc)₂ (10 mg/g or 7.5 mg/g of ARF) revealed similar observations and catalytic activity (Figure 5).

### Example 7: Catalytic Activity: Heck coupling reaction

The catalytic activity of ARF-Pd was first examined in the Heck coupling reactions. As a first model case, we chose the reaction of 3-chloroiodobenzene with ethyl acrylate. The Heck coupling between these two coupling partners is known to give high yields of the coupled product in presence of both homogeneous and other heterogeneous palladium catalysts. Using our heterogeneous catalyst, ARF-Pd, and carrying out the reaction in presence of triethylamine and toluene, we were able to isolate *trans*-ethyl-3-chlorocinnamate in 86% yield. The *trans-*stereochemistry of the double bond was assigned on the basis of the value of the coupling constant (*J* = 15.9 Hz). As observed in some reports (L. Djakovitch, K. Köhler, J. Am. Chem. Soc.2001, 123, 5990-5999), we did not see any other products in this reaction when characterized by NMR. The product was isolated in pure form simply by filtering off the resin-bound catalyst followed by chromatographic purification of the concentrated residue. Similar reactions were conducted with other substituted iodoarenes and the results are summarized in Table 2. In each case, the NMR spectra indicated *trans* double bonds of 2-aryl acrylates, the *J* values range from 15.6-16.2 Hz. Interestingly, the bromo-substituent remained unchanged, which is typically seen for other halogens like chloro or fluoro groups on the aromatic moiety, thereby allowing more selectivity in Heck coupling reaction. The *ortho*-substituent did not cause any steric inhibition, as usually experienced in other coupling reaction, which may be attributed to high activity of the catalyst. In the case of 3-iodobenzene, both the mono- and bis-coupled products were isolated in the ratio of 1:2, when performed the reaction at 100°C for 7 h.

**Representative Procedure for Heck Reaction:** To a suspension of ARF-Pd (300 mg, 2 mol% Pd) in toluene (2 mL) were added 4-bromo-1-iodo anisole (313 mg, 1 mmol), ethyl acrylate (220 mg, 2.2 mmol), triethyl amine (202 mg, 2 mmol) and the reaction mixture was heated under N₂ with gentle magnetic stirring at 90 °C for 10 h. The reaction mixture was passed through a cotton bed to isolate the catalyst and washed it with dichloromethane. The combined washings were concentrated under reduced pressure leaving a residue, which was purified by column chromatography on silica gel. Elution with ethyl acetate/light petroleum (1:10) afforded ethyl-3-(5-bromo-2-methoxyphenyl) acrylate as color less solid (256 mg, 90% yield); m.p.63-64 °C; IR (Nujol): νₘₐₓ 1713, 1632, 1589 cm⁻¹ ; ¹H NMR (300 MHz, CDCl₃, 300K): δ=7.87 (d, 1H, *J* = 16.2 Hz); 7.59 (d, 1H, *J* = 2.4 Hz); 7.40 (dd, 2H, *J* = 8.7 & 2.4 Hz); 6.77 (d, 1H, *J* = 9 Hz); 6.48 (d, 1H, *J* = 16.2 Hz), 4.26 (q, 2H, *J* = 7.2 Hz); 3.85 (s, 3H); 1.33 (t, 3H, *J=* 7.2). ¹³C NMR (75 MHz, CDCl₃, 300K): δ=166.9, 157.1, 138.2, 133.6, 131.0, 125.3, 119.8, 113.1, 112.8, 60.8, 55.6, 14.2.

**Table 2: Heck Coupling of aryl iodides with ethyl acrylate using ARF-Pd.**

| Entry | Haloaromatics | Conditions^{[a]} Temp./ Time | Product(s) | Yield [%]^{[b]} | |
|---|---|---|---|---|---|
| 1 | 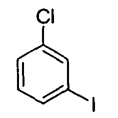 | 90°C/ 5h | 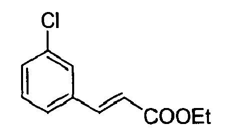 | 86 | |
| 2 | 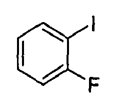 | 90°C/ 5h | 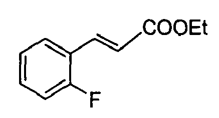 | 81 | |
| 3 | 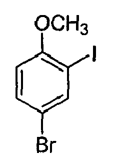 | 90°C/10h | 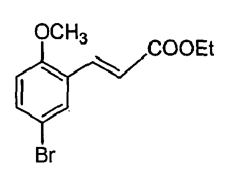 | 90 | |
| 4 | 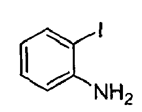 | 95°C/ 9h | 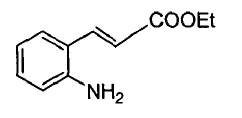 | 73 | |
| 5 | 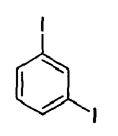 | 100°C/ 7h | 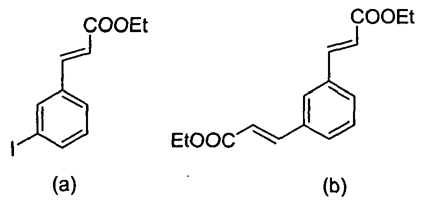 | 30 (a) | 65 (b) |

| | | | | | |
|---|---|---|---|---|---|
| [a] The reactions were carried out in toluene as the solvent; ARF-Pd was used 300 mg per mmol of the bromoarene. [b] Yields are of isolated pure compounds, characterized by IR and NMR spectral data. | | | | | |

### Example 8: Suzuki-Miyaura coupling reaction

The Suzuki-Miyaura coupling reaction between bromoarenes and aryl boronic acid is considered as one of the most important methods for preparing biaryls ( a) Organopalladium Chemistry for Organic Synthesis (Ed.: E.-i. Negishi), Wiley-Interscience, New York, 2002; b) Metal-Catalyzed Cross-Coupling Reactions(Eds.: A. de Meijere, F. Diederich), Wiley-VCH, Weinheim, 2004; c) C. C. Mauger, G. A. Mignani, Aldrichimica Acta.2006, 39, 17-24; d) W. A. Herrmann, K. Öfele, D. v. Preysing, S. K. Schneider, J. Organocmet. Chem. 2003, 687, 229-248. e)For review on recent applications of Suzuki-Miyaura coupling, see: S. Kotha, K. Lahiri, D. Kashinath, Tetrahedron2003, 58, 9363-9695; f) For review on Heck coupling, see: I. P. Beletskaya, A. V. Cheprakov, Chem. Rev.2000, 100, 3009-3066 ). While discovery of this reaction dates back to over twenty five years, present interest is primarily associated with the development of the more potential and versatile catalysts. The use of highly active catalysts at low loadings not only minimizes the amount of palladium for reasons of cost but also offers advantages to put minimum efforts to obtain the products free from the catalyst. On attempting the Suzuki-Miyaura reaction with ARF-Pd, we first conducted coupling of 4-bromo-2-methyl anisole with phenyl boronic acid using the catalyst and base in DMF. Heating the mixture at 110°C for 5 h under N₂ followed by removal of ARF-Pd by filtration and chromatographic purification of the residue furnished the desired unsymmetrical biphenyl in 82% yield. High activity of the catalyst coupled with our interest in multi-couplings in one-pot reaction (B. Basu, P. Das, Md. M. H. Bhuiyan, S. Jha, Tetrahedron Lett.2003, 44, 3817-3820) we put our attention to evaluate the activity of ARF-Pd in one-pot sequential couplings of di- and tri-bromoarenes. Indeed, we were able to prepare bis-and tris-coupled aryl benzenes in good to excellent yields, showing notable activity of the heterogeneous Pd-catalysts. In the case of 1,2-dibromobenzene, we were previously unsuccessful in bis-couplings in one-pot reaction using Pd(OAc)₂ as the source of palladium. The high activity of ARF-Pd made the coupling reactions successful thereby yielding the bis-product (ortho-terphenylene) in 55% yield. Similarly, 9,10-dibromoanthracene afforded the corresponding diphenylanthracene in 90% yield. The results are shown in Table 3.

**Representative Procedure for Suzuki-Miyaura Reaction:** A mixture of 1,3-Dibromobenzene (236 mg 1.0 mmol), phenylboronic acid 271 mg (2.2 mmol) and sodium carbonate (424 mg, 4 mmol) was taken in DMF (2 ml) and then added ARF-Pd (300 mg, 2 mol% Pd). The mixture was heated under nitrogen at 110°C for 2.5 h. After cooling, the reaction mixture was diluted with 10ml of cold water and filtered through a cotton bed. The filtrate was extracted with ether (3 x 30 ml) and the combined organic layer was dried over anhydrous Na₂SO₄. Removal of the solvent left a solid residue, which was purified by column chromatography on silica gel (light petroleum) to give *m*-terphenyl as color less solid (193 mg, 84% yield), m.p. 87-88 °C (Lit.³⁰ m. p. 89°C); IR (Nujol): νₘₐₓ 1454, 1377 cm⁻¹ ; ¹H NMR (300 MHz, CDCl₃, 300K): δ=7.78 (s, 1H), 7.62- 7.28 (m, 13H), ¹³C NMR (75 MHz, CDCl₃, 300K): δ=141.7, 141.1, 129.1, 128.7, 127.3, 127.20, 126.1.

**Table 3: Suzuki-Miyaura coupling between aryl bromide and aryl boronic acid using ARF-Pd.**

| Entry | Haoloaromatics | Conditions^{[a]} Temp./ Time | Products | Yield [%]^{[b]} |
|---|---|---|---|---|
| 1. | 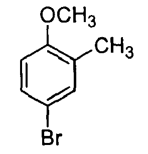 | 110°C/ 5h | 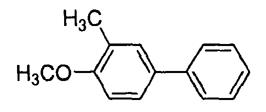 | 82 |
| 2. | 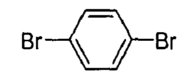 | 110°C/ 5h | 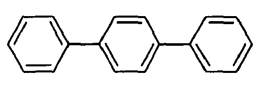 | 84 |
| 3. | 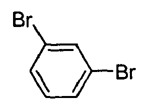 | 110°C/ 2.5h | 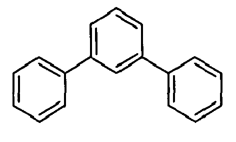 | 84 |
| 4. | 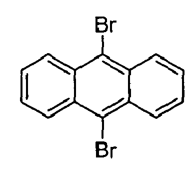 | 115°C/ 8h | 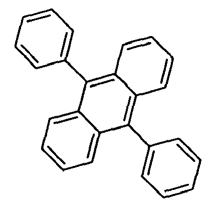 | 90 |
| 5. | 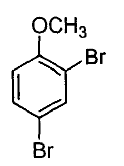 | 110°C/ 5h | 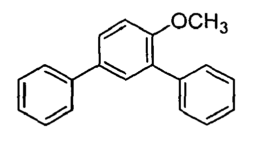 | 86 |
| 6 | 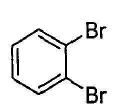 | 110°C/ 6h | 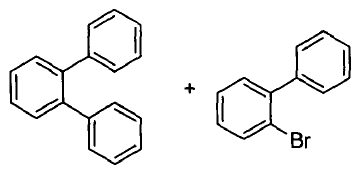 | 55 & 30 |
| 7 | 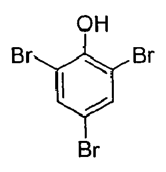 | 120°C/ 6h | 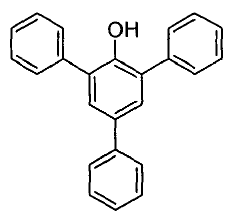 | 62 |

| | | | | |
|---|---|---|---|---|
| [a] The reactions were carried out in DMF; ARF-Pd (300 mg) was taken for 1 mmol of the bromoarenes. [b] Yields are of isolated products, characterized by m.p.; IR and NMR spectral data. | | | | |

### Example 9: Buchwald-Hartwig (C-N) coupling reactions

Over the last decade, the Pd-catalyzed C-N coupling reactions, developed independently by Buchwald and Hartwig, has become a versatile tool for preparing aryl amines from aryl halides, triflates etc (For reviews: a) J. P. Wolfe, S. Wagaw, J. F. Marcoux, S. L. Buchwald, Acc. Chem. Res.1998, 31, 805-818; b) A. R. Muci, S. L. Buchwald, Top. Curr. Chem.2002, 219, 133-209; c) J. F. Hartwig, Pure Appl. Chem.1999, 71, 1417-1423; d) J. F. Hartwig, Acc. Chem. Res. 1998, 31, 852-860. ). While employing similar coupling with halopyridines, Buchwald had experienced difficulty in achieving high yield of the product, possibly because of the formation of stable palladium complexes with pyridine. Such problem of trapping Pd out of the catalytic cycle was, however, circumvented by using chelating bis-phosphine ligands and the amination of bromopyridines was accomplished successfully with Pd₂dba₃-BINAP and other such catalytic combinations (a) S. Wagaw, S. L. Buchwald, J. Org. Chem. 1996, 61, 7240-7241; b) J. Wolfe, S. L. Buchwald, J. Org. Chem.2000, 65, 1144-1157). Since aminopyridines are versatile intermediates as drugs and dyes, we were interested to apply our resin-bound palladium catalyst in the amination of bromopyridines. We previously reported that KF/alumina could serve as a potential basic surface for amination reaction under solvent-free conditions (a) B. Basu, S. Jha, N. K. Mridha, M. H. Bhuiyan, Tetrahedron Lett. 2002, 43, 7967-7969 ; b) B. Basu, P. Das, A. K. Nanda, S. Das, S. Sarkar, Synlett 2005, 1275-1278). We first examined coupling of 2-bromopyridine with pyrrolidine on KF/alumina surface in presence of ARF-Pd. It was encouraging to observe completion of reaction by heating the reaction mixture at 95°C for 2 h on KF/alumina surface. After the reaction, the solid mixture was simply placed over a column of silica gel to isolate the desired product in 90% yield. Similar reactions were performed with several dibromopyridines and amines using ARF-Pd as the catalyst. In all the cases studied, we obtained the mono-aminated products selectively. It is significant that no ligands (phosphines) are required for the amination, a major feature for industrial applications. The results are summarized in Table 4.

**Representative Procedure for Amination of Halopyridines:** 2,5-Dibromopyridine (237 mg, 1 mmol) and 1 gm of KF/Al₂O₃^{[28]} were mixed under nitrogen, then ARF-Pd (300 mg, 2 mol% Pd) and 4-benzyl piperidine ( 486 mg, 3 mmol) were added to it. The final mixture was covered under N₂ and placed on pre-heated oil bath at 90 °C for 2.5 h. After cooling to room temperature, the solid mixture was transferred on a column of silica gel and elution with ethyl acetate/light petroleum (1:49) afforded 2-(4-benzylpiperidin-1-yl)-5-bromopyridine (291 mg, 88%); M.p. 78 °C; IR (Nujol): νₘₐₓ 1582, 1543, 1472 cm⁻¹; ¹H NMR (300 MHz, CDCl₃, 300K): δ = 8.16 (d, 1H, *J* = 2.4 Hz), 7.47 (dd, 1H, *J* = 9 & 2.4 Hz), 7.31- 7.13 (m, 5H), 6.51 (d, 1H, *J* = 9 Hz), 4.22- 4.18 (m, 2H), 2.80- 2.70 (m, 2H), 2.55 (d, 2H, *J* = 6.9 Hz), 1.82- 1.69 (m, 3H), 1.31- 1.18 (m, 2H). ¹³C NMR (75 MHz, CDCl₃, 300K) δ=157.9, 148.4, 140.2, 139.6, 129.1, 128.2, 125.9, 108.5, 106.7, 45.7, 43.1, 38.2, 31.6.

**Table 4: Buchwald-Hartwig amination of bromopyridines with sec. amines using ARF-Pd.**

| Entry | Halopyridine | Amine | Conditions^{[a]} Temp./ Time | Product | Yield [%]^{[b]} |
|---|---|---|---|---|---|
| 1 | 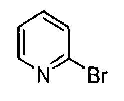 | 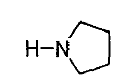 | 95°C/ 2h | 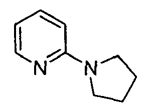 | 90 |
| 2 | 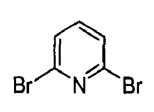 | 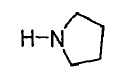 | 90°C/ 1h | 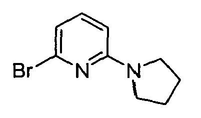 | 86 |
| 3 | 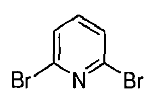 | 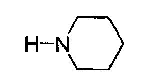 | 80°C/ 2h | 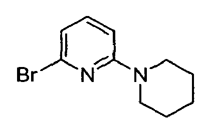 | 77 |
| 4 | 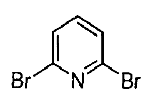 | 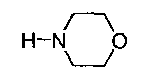 | 90°C/ 1h | 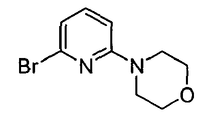 | 79 |
| 5 | 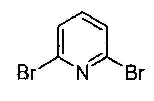 | 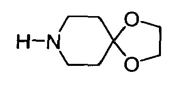 | 80°C/ 2.5h | 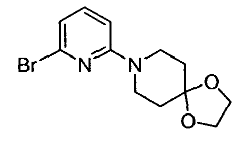 | 81 |
| 6 | 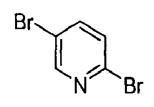 | 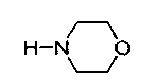 | 90°C/ 2h | 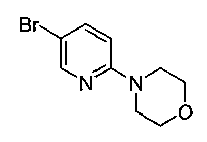 | 86 |
| 7 | 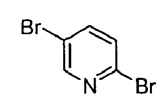 | 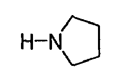 | 80°C/ 2h | 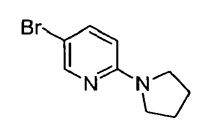 | 92 |
| 8 | 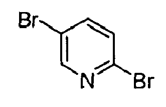 | 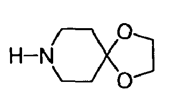 | 90°C/ 3h | 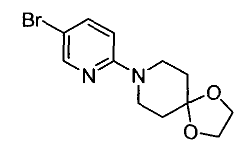 | 84 |
| 9 | 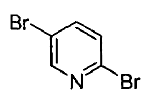 | 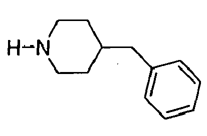 | 90°C/ 2.5 h | 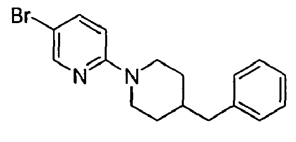 | 88 |

| | | | | | |
|---|---|---|---|---|---|
| [a] The reactions were carried out using KF/alumina and without any solvent; ARF-Pd (300 mg) per mmol of bromopyridine. [b] Yield of isolated products, charaterized by m.p., IR and NMR spectral data. | | | | | |

### Example 10: Minimum concentration of Palladium on the surface required for efficient catalytic activity

In order to find the minimum atomic concentration of palladium that is required to exhibit efficient catalytic activity, we chose the Suzuki-Miyaura coupling reaction as the model case. Different amounts of palladium acetate (1-10 mg) per 1 g of the ARF were used to immobilize palladium under various conditions and the resulting ARF-Pd was used in the Suzuki-Miyaura coupling of 1,4-dibromobenzene with phenylboronic acid. Loading of palladium from Pd(OAc)₂ used up to 5 mg for 1 g of ARF seemed to decrease the catalytic efficiency in the second run of Suzuki-Miyaura coupling. Repeating the process of immobilization at different temperatures did not result any significant enhancement in catalytic activity. In fact, immobilization of palladium using either 10 mg of Pd(OAc)₂ for 1 g of ARF at 50°C for 3 h did show excellent catalytic activity even up to the fifth run with the formation of the coupled product in 80-84% yields. While probing the leaching of palladium from the surface, it appeared from XPS studies that in the first-run of Suzuki-Miyaura coupling there was a tendency of palladium to leach out. Interestingly, the leaching effect came to a steady state from the second run. We therefore performed another study taking 7.5 mg of Pd(OAc)₂ for 1 g of ARF. The resulting ARF-Pd was again taken in DMF and stirred gently at 100°C for 2 h. After usual washing and drying, the resin beads of ARF-Pd were used in Suzuki-Miyaura coupling reaction and found its catalytic activity up to three runs in an acceptable range. The results are summarized in Table 5. The minimum effective concentration of Pd in the surface of the resin was established as the use of 7.5 mg of Pd(OAc)₂ for 1 g of the ARF and prepared at 50°C for 3 h in DMF followed by treatment of the ARF-Pd in DMF at 100°C for 2 h.

**Table 5 Minimum concentration of Palladium from Pd(OAc)₂**

| Entry | Pd(OAc)₂ | ARF | Temp./ Time/ Conditions^{[a]} | %Yield in Suzuki - Miyaura coupling product^{[b]} |
|---|---|---|---|---|
| 1. | 10.0mg | 1g | 1 h at rt and then 50°C /3h | 84% (1^{st} Run) 84% (2^{nd} Run) 81% (3^{rd} Run) 80% (4^{th} Run) 80% (5^{th} Run) |
| 2^{[c]} | 7.5mg | 1g | 1 h at rt and then 50°C /3h, then filtered, washed and dried. Again taken in DMF, heated at 100°C for 2 h, and filtered, washed and dried. And used in Suzuki Coupling reaction | 83% (1^{st} Run) 81% (2^{nd} Run) 78% (3^{rd} Run) |
| 3. | 5mg | 1g | 1 h at rt and 50°C /2h | 78% (1^{st} Run) 50% (2^{nd} Run) |
| 4.^{[c]} | 5mg | 1g | 1 h at rt and 70°C /2h | 78% (1^{st} Run) 35% (2^{nd} Run) |
| 5. | 2.5mg | 1g | 1 h at rt and 50°C /2h | 76% (1^{st} Run) 46% (2^{nd} Run) |
| 6.^{[d]} | 2.5mg | 1g | 1 h at rt and 50°C /2h, then filtered and dried. Again takenin DMF, heated at 100°C for 1h, filtered and dried. | 76% (1^{st} Run) 46% (2^{nd} Run) |
| 7. | 1.0mg | 1g | 1 h at rt and 50°C /2h, | < 5% (1^{st} Run) |

| | | | | |
|---|---|---|---|---|
| [a] Occasional shaking the suspension of ARF and Pd(OAc)2 in DMF. [b] SM reaction was studied between the coupling partners: 1,4-dibromobenzene (1 mmol) and phenylboronic acid (2.5 mmol). [c] To observe any change in catalytic activity when immobilization is performed at higher temperature. [d] To examine any leaching of Pd before applying in reaction. | | | | |

### Example 11: Recycle experiments and leaching of palladium

Recycle experiments were also verified taking the Suzuki-Miyaura couplings between 1,4-dibromobenzene and phenylboronic acid. After the reaction, the catalyst (ARF-Pd) was filtered off, washed with dichloromethane, water and finally dried under vacuum for further use. Consecutive five runs were tested to evaluate the activity of ARF-Pd that was prepared from 10 mg of Pd(OAc)₂ for 1 g ARF (Table 5, Entry 1). The ARF-Pd prepared from 7.5 mg of Pd(OAc)₂ for 1 g ARF (Table 5, Entry 2) was also tested for three run with almost equal efficiency. The results are presented in Table 6, which show that the catalyst is remarkably active even in the fifth run /third run and thus the coupled product, *p*-terphenylene, was isolated without any significant drop of yields.

**Table 6: Recycling Experiments in Suzuki-Miyaura Couplings using ARF-Pd.**

| | | | | | |
|---|---|---|---|---|---|
| 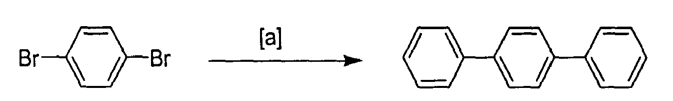 | | | | | |
| Run | 1 | 2 | 3 | 4 | 5 |
| Yield [%] | 84 (83)^{[b]} | 84 (81) | 81 (78) | 80 | 80 |
| Time [h] | 5 | 5 | 5 | 5 | 5 |

| | | | | | |
|---|---|---|---|---|---|
| [a] Reagents & Conditions: 1,4-Dibromobenzene (2 mmol), Phenylboronic acid (4.5 mmol), ARF-Pd^{*} = 600 mg, DMF (4 mL), 110°C. ^{*}ARF-Pd prepared from 10 mg of Pd(OAc)₂ for 1 g ARF (Entry 1, Table 5). [b] Yields in the parenthesis represent use of ARF-Pd prepared from 7.5 mg of Pd(OAc)₂ for 1 g ARF (Entry 2, Table 5). | | | | | |

Leaching of palladium should generally depend on the nature of solvent used for a reaction. Although there is evidence that toluene as the solvent could prevent leaching of palladium, the poor solubility of the coupling partners and the extrinsic base (Na₂CO₃) in toluene might result in poor yield of the product (M. Dams, L. Drijkoningen, B. Pauwels, G. Van Tendeloo, D. E. De Vos, P. A. Jacobs, J. Catal. 2002, 209, 225-236). We therefore carried out the Suzuki-Miyaura couplings between 1,4-dibromobenzene and phenylboronic acid in DMF as the solvent. The reaction was repeated using the recovered ARF-Pd and its palladium content was measured by XPS studies. In fact, leaching of palladium was followed by monitoring the change in Pd/N atomic ratio at the surface of catalyst after each cycle. The results show that the palladium content on surface seems to reach its steady-state after the second catalytic run and its catalytic activity remains notable up to the fifth run investigated. Detectable decrease in Pd/N atomic ratio may be caused by Pd leaching due to re-structuring of active particles during the course of the reaction.

Although the mechanism of involving Pd⁽⁰⁾ species is generally accepted in homogeneously catalyzed coupling reactions, such thought for heterogeneous catalysts still remains under consideration. Choudary et al. have recently reported that the surface bound Pd⁽⁰⁾ is the active species, analogous to homogeneously catalyzed coupling reactions, which undergoes oxidative addition to aryl C-halogen bonds (B. M. Choudary, S. Madhi, N. S. Chowdari, M. L. Kantam, B. Sreedhar, J. Am. Chem. Soc. 2002, 124, 14127-14136; B. M. Choudary, S. Madhi, M. L. Kantam, B. Sreedhar, Y. Iwasawa, J. Am. Chem. Soc. 2004, 126, 2292-2293). As observed in XPS studies of ARF-Pd, the Pd spectra suggests the presence of both Pd⁽⁰⁾ and Pd^{(II)} species at the polymer surface. At this stage, we propose that the Pd⁽⁰⁾ is the active species, having soaked at the resin surface possibly through reduction of Pd^{(II)} salts by the carboxylate functions (formate) and that the Pd^{(II)} species, which might come out of the surface during the reactions showing a low level leaching of palladium in the second catalytic run. No further leaching is observed in subsequent runs as the Pd/N ratio seems to attain a steady-state after the second catalytic run (Figure 6).

In summary, we have demonstrated for the first time that suitably designed ion-exchange resins can be used as the potential polymeric surface to scavenge and immobilize palladium metal and the resulting palladium-soaked ionic resins can be used as efficient heterogeneous catalyst. The major advantage of using the formate as the counter anion is to facilitate reduction of Pd(II) to Pd(0) in situ followed by deposition on the surface. The heterogeneous Pd-catalyst exhibits high catalytic efficiency in Heck, Suzuki-Miyaura (C-C bond-formimg) and Buchwald-Hartwig (C-N bond-forming) reactions. The phosphane or ligand-free reaction conditions could be of potential interests for commercial applications of heterogeneous catalyst in C-C and C-N coupling reactions. Strict compliance with absence of air or moisture is not required and easy separation of the catalyst, isolation of the products in good to excellent yields and reuse of the ARF-Pd for at least five runs are attractive facets for the heterogeneous catalyst. The resin soaked catalyst can be stored for several weeks without any special protective arrangements like under-argon atmosphere or low temperatures.

## Claims

1. A method of producing a heterogeneous catalyst suitable for catalyzing Heck, Suzuki-Miyaura and Buchwald-Hartwig reactions, comprising the steps of:
a) providing a porous carrier, said porous carrier consisting of a core and a plurality of ion exchange groups covalently bonded to the surface of said core, where at least 90 % of the ions bound to said carrier are formate ions;
b) providing a palladium (II) salt;
c) suspending said carrier in an organic solvent thereby obtaining a suspension;
d) adding said palladium salt to said suspension and allowing the resulting mixture to react at a temperature within the range of 30 - 70 °C until said carrier has turned black;
e) washing said carrier in water; and
f) drying said carrier under vacuum;
**characterised in that**
g) the resulting carrier subsequently is resuspended in dimethyl formamide and heated under inert atmosphere to at least 90 °C for 2 hours; followed by
h) washing with a polar solvent; and
i) drying.

2. A method according to claim 1, **characterised in that** the resulting carrier after step i) is allowed to catalyse a chemical reaction chosen from the group of Heck, Suzuki-Miyaura and Buchwald-Hartwig reactions.

3. A method according to anyone of claims 1 - 2, **characterised in that** the carrier has the shape of spherical particles having a size ranging from 10 µm to 2 mm and a porosity from 50 to 1000 Å, or a monolithic structure.

4. A method according to anyone of claims 1 - 3, **characterised in that** the core of said carrier is made of a material chosen from the group of porous silica, zirconium, graphite, or a polymer or copolymer chosen from the group of mono- or oligovinyl monomer units, such as styrene and its substituted derivatives, acrylic acid or methacrylic acid, alkyl acrylates and methacrylates, hydroxyalkyl acrylates and methacrylates, acrylamides and methacrylamides, vinylpyridine and its substituted derivatives, divinylbenzene, divinylpyridine, alkylene diacrylate, alkylene dimethacrylate, oligoethylene glycol diacrylate and oligoethylene glycol dimethacrylate with up to 5 ethylene glycol repeat units, alkylene bis(acrylamides), piperidine bis(acrylamide), trimethylolpropane triacrylate, trimethylolpropane trimethacrylate, pentaerythriol triacrylate and tetraacrylate, and mixtures thereof.

5. A method according to claim 4, **characterised** that the carrier is an Amberlite ion exchange bead.

6. A method according to anyone of claims 1 - 4, **characterised in that** ion-exchange groups are introduced on the surface of said carrier before step c) in the method of claim 1 by functionalizing via a chemical reaction.

7. A method according to claim 6, **characterised in that** the ion-exchange groups have been introduced to the carrier by polymerising, preferably graft polymerising , monomers comprising ion-exchange groups onto the surface of the carrier core.

8. A method according to anyone of claim 1 - 7, **characterised in that** the ion-exchange groups are a type 1 or a type 2 quaternary ammonium group.

9. A method according to anyone of claims 1 - 8, **characterised in that** at least 95%, and preferably at least 99% of the ions bound to the carriers are formate ions.

10. A method according to anyone of claims 1 - 9, **characterised in that** said palladium (II) salt is chosen from the group of palladium acetate (Pd(CH₃COO)₂) and disodium palladium tetrachloride (Na₂PdCl₄).

11. A method according to anyone of claims 1 - 10, **characterised in that** said organic solvent in step c) is chosen from the group of dimethyl formamide (DMF), acetonitrile, toluene and dioxane.

12. A method according to anyone of claims 1 - 11, **characterised in that** the resulting mixture is allowed to react at a temperature within the range of 40 - 60 °C, and in particular within the range of 45 - 55 °C.

13. A method according to anyone of claims 1 - 12, **characterised in that** the polar solvent used in the final washing is chosen from the group of dimethyl formamide, water dichloromethane, acetone, diethyl ether, tetrahydrofuran and dioxane

14. A heterogeneous catalyst comprising a porous carrier consisting of a core and a plurality of ion exchange groups covalently bonded to the surface of said core on which surface Pd (0) as well as the formate anion has been deposited, **characterized in that** the catalyst has been produced by a method according to anyone of claims 1 - 13.

15. A reactor comprising a hollow tube, said tube having two open ends, **characterised in that** the reactor contains a heterogeneous catalyst according to claim 14.

16. Use of a heterogeneous catalyst according to claim 14 for catalyzing Heck, Suzuki-Miyaura and Buchwald-Hartwig reactions.
